# FASCICULE DE BREVET EUROPEEN

(11) **EP 0 839 141 B1**
(45) Date de publication et mention de la délivrance du brevet: **26.09.2001**
(21) Numéro de dépôt: 96924941.6
(22) Date de dépôt: 05.07.1996
(51) Int. Cl.: C07D 305/14

(54) **PROCEDE D'EXTRACTION ET D'ISOLEMENT DE LA 10-DESACETYLBACCATINE III**
VERFAHREN ZU EXTRAHIEREN UND ISOLIERUNG VON 10-DESACETYLBACHATIN III
METHOD FOR EXTRACTING AND ISOLATING 10-DEACETYLBACCATIN III

(30) Priorité: 07.07.1995 FR 9508241
(43) Date de publication de la demande: 06.05.1998
(73) Titulaire: Pierre Fabre Médicament, 81106 Castres Cedex (FR)
(72) Inventeur: NEGOL, Paul, F-81290 Labruguière (FR); SERRANO, Marie-Jeanne, 140, route de Terssac F-81000 Albi (FR); DAVID, Bruno, F-31650 Saint-Orens-de-Gameville (FR)
(74) Mandataire: Ahner, Francis
(86) Numéro de dépôt international: FR9601050
(87) Numéro de publication internationale: WO9703061

(56) Documents cités:
- WO-A-95/17395
- FR-A- 2 696 462
- FR-A- 2 696 463

## Description

La présente invention concerne un procédé d'extraction et d'isolement de la 10-désacétylbaccatine III, composé utile comme intermédiaire dans l'hémisynthèse du taxol ou de ses dérivés présentant un intérêt oncologique.

Les taxanes, dont le taxol, sont des agents anticancéreux aux propriétés particulièrement intéressantes.

Toutefois, les taxanes naturels anticancéreux sont principalement présents en faible concentration dans les écorces d'if, végétaux connus pour la finesse de leur écorce et leur croissance très lente.

Afin d'assurer une exploitation rentable des taxanes anticancéreux sans porter préjudice à la source végétale de matières premières, il a été rapidement envisagé d'obtenir ces produits par hémisynthèse à partir de la 10-désacétylbaccatine III ou tétraol.

En effet, ce dernier se trouve en quantité plus importante dans les feuilles d'if, comme l'ont montré Sénilh & al. en 1981 (C. R. Acad. Sc. Paris, 1981, 293 (2), 501-503), assurant une source renouvelable de matière première dans la préparation du médicament.

Le tétraol a été décrit la première fois comme le produit résultant de la méthanolyse alcaline du taxol par M.C. WAN1 en 1971 lors de la mise en évidence du taxol.

L'obtention de ce produit avec un rendement élevé deviènt donc un des aspécts essentiels du problème de l'exploitation rentable des taxanes d'intérêt oncologique comme le taxol et le taxotère,

Or, si le principe général d'extraction des taxanes à partir des feuilles d'if est connu, peu de procédés concernent spécifiquement le tétraol.

En effet, le but de la quasi-totalité des procédés est d'obtenir le taxol, produit à haute valeur ajoutée, alors que le tétraol est toujours pris en considération comme sous-produit d'extraction.

Il s'agit en général d'extraire le végétal par un alcool, puis d'adopter les conditions de purification spécifiques à l'obtention du taxol.

Cependant, les demandes de brevet EP-A-0 253 738 et EP-A-0 253 739 mentionnent un procédé d'extraction du tétraol qui consiste à reprendre un extrait éthanolique des feuilles d'if par de l'eau, le filtrer sur Célite et à extraire le filtrat par le chlorure de méthylène avant de l'extraire par l'eau puis de séparer les différentes fractions. Le tétraol est obtenu sous forme purifiée après deux chromatographies successives sur colonne de silice.

Ce procédé utilisé dans les deux demandes de brevet citées ci-dessus est donc très long et complexe.

Les feuilles non séchées sont extraites par de l'éthanol à 95° (cinq macérations de dix heures chacune). Les extraits alcooliques sont réunis et concentrés afin d'éliminer l'éthanol résiduel. La suspension aqueuse est reprise par du'chlorure de méthylène (neuf extractions). L'extrait dichloro-méthylénique est concentré puis chromatographié sur colonne de silice. La fraction riche en tétraol est à nouveau soumise à une chromatographie sur colonne de silice. Le rendement d'extraction n'est pas mentionné et le titre du produit final est supposé à 100 %.

La demande de brevet WO-A-92/07842 décrit un procédé où intervient une préparation par chromatographie en phase inverse. L'extrait brut d'écorces ou de feuilles de Taxus est traité par chromatographie en phase inverse ce qui permet d'adsorber les taxanes qui seront élués sélectivement. Les phases inverses citées sont la silice C₈, la silice CN et la silice C₁₈.

La demande de brevet WO-A-92/18492 décrit un procédé général d'extraction et de purification des taxanes notamment par adsorption/élution et par extraction liquide/liquide.

La plante est extraite par un solvant organique (éthanol, acétone, acétate d'éthyle). La phase organique est reprise par le dichlorométhane ou acétate d'éthyle et les fractions hydrophiles sont extraites par de l'eau. La phase organique est reprise par un mélange acétate d'éthyle/méthanol (3/1) pour adsorber l'extrait sur Célite. La Célite éluée par du dichlorométhane conduit à un extrait de taxanes.

Une variante consiste à remplacer la purification par la Célite par une extraction liquide/liquide par l'hexane et un mélange méthanol/eau (9/1).

Ces méthodes dans tous les cas ne conduisent qu'à un mélange brut de taxanes, qui seront ensuite purifiés par des techniques chromato-graphiques traditionnelles.

Dans la demande EP-A-0 521 675 une préparation par CO₂ supercritique de la 10-désacétylbaccatine III est décrite. Cependant le procédé est proposé plus spécialement pour l'extraction du taxol.

Deux procédés spécifiques d'extraction du tétraol décrits dans les demandes de brevet WO-A-94/07881 et WO-A-94/07882, tendent vers une simplification de leur mise en oeuvre, par une purification essentiellement liquide/liquide. Il s'agit d'effectuer une première extraction des feuilles par l'eau ou un alcool, avec dans ce cas l'ajout d'eau à la phase alcoolique obtenue, puis de concentrer la phase aqueuse, et de l'extraire par un solvant organique tel que des éthers ou esters aliphatiques. Après évaporation du solvant, le résidu solide enrichi en tétraol est purifié par cristallisation sélective dans un solvant approprié, en particulier l'acétonitrile.

Un tel procédé, bien que simplifié comprend néanmoins un grand nombre d'étapes indispensables, coûteuses en solvant et en rendement, que l'on peut résumer comme suit :
- première étape d'extraction des feuilles d'if par de l'eau ou un alcool pour obtenir une phase aqueuse contenant le tétraol, ladite phase aqueuse étant un extrait initial résultant directement de la première étape d'extraction par l'eau ou étant obtenue après ajout d'eau après extraction par l'alcool,
- deuxième étape d'extraction de ladite phase aqueuse par un solvant organique approprié essentiellement non miscible à l'eau, de manière à obtenir une phase organique riche en tétraol,
- troisième étape d'évaporation du solvant organique pour obtenir un résidu solide enrichi en tétraol, puis
- dernière étape d'isolement et de purification du tétraol à partir du résidu solide par cristallisation sélective dans un solvant de cristallisation.

WO-A-95/17395 décrit également un procédé d'extraction du tétraol à partir de feuilles d'if consistant en une première extraction des feuilles d'if par un solvant polaire protique, puis en une deuxième extraction de la phase aqueuse ainsi obtenue par un solvant organique approprié essentiellement non miscible à l'eau et en une étape d'isolement du tétraol à partir de la phase organique précédemment obtenue. Cependant, la mise en oeuvre de ce procédé nécessite une "mise à sec" de l'extrait enrichi en tétraol juste avant l'étape de cristallisation.

Il est donc important de chercher à simplifier encore les procédés d'extraction du tétraol, de manière à faciliter leur exploitation industrielle, permettant de diminuer l'usage de solvants organiques et leur retraitement ultérieur, tout en conservant de bons rendements d'obtention d'un extrait à haute teneur en tétraol.

La présente invention concerne donc un procédé amélioré et simplifié d'extraction et d'isolement du tétraol à partir des feuilles d'if.

Les végétaux utiles pour le procédé selon l'invention sont de préférence les espèces du genre Taxus (if). Certains botanistes différencient le genre Taxus en plusieurs espèces : T. baccata, T. brevifolia, T. wallichiana, T. chinensis, T. cuspidata, T. canadensis, T. floridana, T. globosa, T. media, T. hunnewelliana ... alors que d'autres auteurs considèrent toutes ces espèces comme des sous-espèces géographiques d'une espèce collective : T. baccata. Le procédé s'applique donc sur l'espèce collective T. baccata au sens large ainsi que sur les cultivars correspondants.

Le procédé selon l'invention étant spécifique à l'extraction et l'isolement du tétraol, c'est bien par rapport à ce produit que seront déterminées les conditions spécifiques de sa mise en oeuvre.

De ce fait, bien que spécifié pour l'extraction et la purification du tétraol à partir des feuilles d'if, le procédé selon l'invention pourra très bien s'adapter à tout milieu riche en taxanes et plus particulièrement en taxanes de polarité voisine du tétraol, quelle que soit son origine, naturelle ou non, végétale ou non, comme des cultures cellulaires d'écorces ou de feuilles d'if, des cultures de champignons ou leurs cultures cellulaires, ou encore des mélanges de taxanes, résidus d'extraction du taxol.

Les manipulations décrites en exemples ont été réalisées sur des feuilles séchées dont la teneur en tétraol était voisine de 900 mg/kg. Bien entendu, l'invention s'applique également à des Taxus pauvres en tétraol (300 mg/kg) et a fortiori à des Taxus plus riches (1000 mg/kg et au-delà).

Le procédé selon l'invention est caractérisé en ce qu'il consiste à mettre en oeuvre les étapes suivantes :
- première étape d'extraction des feuilles d'if par un solvant polaire protique consistant en un mélange acétone / eau pour obtenir une phase aqueuse concentrée contenant le tétraol,
- deuxième étape d'extraction du tétraol de ladite phase aqueuse par de l'acétonitrile,
- troisième étape de relargage de l'eau résiduelle de la phase acétonitrile par addition d'un solvant chloré,
- dernière étape d'isolement du tétraol par évaporation partielle de la phase organique, cristallisation sélective et filtration.

La simplification du procédé selon l'invention apparait immédiatement puisque l'on supprime une étape intermédiaire d'extraction de la phase aqueuse par un solvant organique approprié essentiellement non miscible à l'eau, puis d'évaporation dudit solvant pour obtenir un résidu solide riche en tétraol. L'extrait initial comprend généralement le tétraol en mélange avec des taxanes de polarité voisine, extraits par un même solvant polaire protique. Le but de la deuxième et de la dernière étape consiste alors, par un solvant organique approprié, à extraire et isoler directement le tétraol de ce mélange par cristallisation sélective.

D'une manière avantageuse, les feuilles d'if sont séchées et/ou broyées. La première étape d'extraction est de préférence effectuée par macération dans le solvant polaire protique pour une durée comprise entre 15 minutes et 6 heures, de préférence 1 heure.

L'extrait initial contenant le tétraol est ensuite récupéré, notamment par filtration.

L'homme du métier saura adapter le temps de macération en fonction du nombre choisi d'opérations d'extraction des feuilles. Ainsi, il pourra effectuer plusieurs macérations de 15 minutes avec de faibles quantités de solvant, ou au contraire effectuer une opération équivalente en une seule étape, additionnant les temps de macération et les volumes de solvant employés dans l'extraction multiple.

Le nombre de macérations sera bien entendu choisi avec pour objectif d'épuiser les feuilles d'if de leur teneur en tétraol présent.

Dans le cas de plusieurs macérations, les extraits de chaque macération sont alors regroupés pour donner l'extrait initial contenant le tétraol.

La première étape d'extraction pourra également être conduite d'une manière avantageuse par macération dans le solvant polaire protique sous irradiation de micro-ondes ou d'ultra-sons pendant une durée comprise entre 2 et 15 minutes.

Les feuilles d'if sont extraites à une température comprise entre 10°C et la température d'ébullition du solvant polaire protique, la température préférée étant la température ambiante, que l'on définit dans la présente demande de brevet comme la température du laboratoire, comprise entre environ 15°C et environ 25°C. Bien entendu, il est compris qu'en fonction des variations saisonnières de température, celte température ambiante pourra être inférieure ou supérieure à la fourchette proposée. En fait, on définit plus simplement la température ambiante par la température de l'environnement dans lequel sera conduit le procédé, sans qu'il ne soit indispensable de fournir une énergie additionnelle au milieu d'extractions pour obtenir des rendements satisfaisants.

D'une manière avantageuse, le solvant plaire protique consistant en un mélange acétone/eau est mis en oeuvre dans un rapport volumique jusqu'à 80/20, avantageusement voisin de 70/30.

La phase aqueuse contenant le tétraol, est alors obtenue par évaporation de la cétone. Cette évaporation se réalise avantageusement avec une concentration simultanée de la phase aqueuse. Cette étape de concentration peut être rendue nécessaire quel que soit le solvant de départ eau, eau / alcool et/ou acétone, pour des raisons pratiques lorsque la phase aqueuse peut atteindre des volumes de plusieurs centaines de litres. On pourra réduire avantageusement le volume de la phase aqueuse dans un rapport pouvant aller jusqu'à 70 (volume initial / volume final = 70).

Le solvant organique approprié permettant l'extraction du tétraol, est, comme indiqué ci-dessus de l'acétonitrile.

La deuxième étape pourra bien entendu être répétée plusieurs fois jusqu'à épuisement de la phase aqueuse en tétraol, les différentes fractions organiques étant alors réunies pour la dernière étape d'isolement du tétraol par évaporation partielle de la phase organique, cristallisation sélective et filtration.

Toutefois, avant cette dernière étape, cette cristallisation sélective est précédée par une étape de séchage de la phase organique et/ou de relargage de l'eau résiduelle selon les méthodes usuelles connues de l'homme du métier. Le relargage de l'eau résiduelle peut être effectué soit en augmentant la polarité de la phase aqueuse par addition de sel, en particulier un halogénure de métal alcalin, de préférence le chlorure de sodium, soit en augmentant le caractère hydrophobe de la phase organique par l'ajout d'une quantité appropriée de solvant de relargage apolaire, c'est-à-dire de polarité inférieure à celle du solvant de cristallisation sélective.

Dans ce second cas, on choisira de préférence un solvant dont la température d'ébullition est inférieure à celle du solvant de cristallisation sélective de manière à éliminer le solvant de relargage par évaporation avant d'effectuer la cristallisation sélective du tétraol. Cette élimination du solvant de relargage pourra d'ailleurs s'accompagner d'une évaporation partielle du solvant de cristallisation sélective. D'une manière avantageuse, le solvant de relargage est un solvant halogéné, de préférence le chlorure de méthylène. Dans le cadre de la présente invention, le procédé comprend une troisième étape de relargage de l'eau résiduelle de la phase acétonitrile par addition d'un solvant chloré.

La phase aqueuse résiduelle relarguée pourra être de nouveau extraite par un solvant de cristallisation sélective défini plus haut, de panière à récupérer le tétraol résiduel qui serait resté en solution dans l'eau.

Ensuite, pour la dernière étape du procédé conforme à l'invention, la solution de tétraol dans le solvant de cristallisation sélective, en particulier dans l'acétonitrile est laissée à reposer à froid, de préférence à une température comprise entre 0°C et la température ambiante, avantageusement inférieure à 10°C, encore plus avantageusement comprise entre 0°C et 5°C, pour laisser précipiter le tétraol en laissant les impuretés dans le surnageant.

Le solide précipité sera alors récupéré par les techniques usuelles de l'homme du métier, notamment par filtration et séchage.

Le séchage de la phase organique peut se faire soit par l'ajout de sel, comme par exemple le sulfate de sodium, soit encore par entraînement azéotropique de l'eau résiduelle, permettant de réduire également le volume de solvant de cristallisation sélective, avant d'effectuer cette cristallisation.

Le séchage de la phase organique et/ou le relargage de l'eau résiduelle pourront être avantageusement précédés par un lavage de la phase organique avec de l'eau, de préférence par un volume équivalent d'eau en une ou plusieurs fois.

D'une manière générale, pour faciliter la manipulation des phases organiques, on cherchera à limiter le volume du solvant de cristallisation sélective, avec ou sans séchage et/ou relargage, cette diminution de volume pouvant aller jusqu'à un rapport volume initial / volume final de 8.

Par volume initial, on entend le volume total des fractions organiques réunies après l'extraction de la phase aqueuse par le solvant de cristallisation sélective.

Par volume final, on entend le volume de solvant de cristallisation sélective dans lequel sera effectué la cristallisation sélective du tétraol, c'est-à-dire après un éventuel lavage et/ou relargage et/ou séchage et/ou concentration de cette phase organique.

Après cette cristallisation sélective, la pureté du tétraol isolé est généralement supérieure à 90 % et le rendement d'extraction par rapport à la quantité de tétraol initialement présent dans le végétal est supérieur à 70 % en masse.

Le tétraol ainsi obtenu pourra être purifié une nouvelle fois par recristallisation dans un solvant approprié, de préférence dans le solvant de cristallisation sélective défini ci-dessus, ou encore un solvant de recristallisation notamment choisi parmi les mélanges (en volume) de solvants suivants :
- chlorure de méthylène / hexane : 1/1 à 1/3
- chlorure de méthylène / acétontrile : 1/1 à 1/5
- chlorure de méthylène / hexane / méthanol : 1/1 /0,1
- acétone / hexane : 1/1 à 1/4.

Le tétraol cristallisé peut être simplement purifié par lavage avec de l'eau, après reprise dans un solvant organique approprié tel que l'acétate d'éthyle. On pourra effectuer un premier lavage par une solution alcaline, par exemple de carbonate de calcium, puis un nouveau lavage par l'eau jusqu'au pH neutre. Après évaporation du solvant organique, le tétraol est généralement obtenu avec une très haute pureté, supérieure à 96 %.

D'autres impuretés colorées pourront également être éliminées par un rinçage à l'eau du tétraol obtenu.

Enfin, le tétraol non cristallisé restant dans les eaux mères de la cristallisation sélective pourra être également cristallisé en reprenant la phase organique résiduelle, surnageant du cristallisat, sur laquelle on effectue une succession d'opérations de lavage par un volume égal d'eau, puis de largage de l'eau résiduelle par l'ajout d'un solvant de relargage apolaire défini plus haut ci d'élimination dudit solvant de relargage.

Les exemples ci-après permettent d'illustrer différentes conditions de mise en oeuvre du procédé selon l'invention.

Nous entendons par feuilles "séchées finement broyées" les sommités feuillées séchées à température ambiante ou à température ne dépassant pas 70°C, d'une granulométrie comprise entre 2 mm et 0,5 mm pour les fragments ligneux (moins de 15 % masse/masse totale) et 2 mm à 100 µm pour les fragments de feuilles.

Le dosage des feuilles est effectué de la manière suivante : la poudre de feuilles (prise d'essai de 50 g prélevée selon les techniques classiques d'échantillonnage) est extraite à deux reprises par 500 ml de méthanol à température ambiante. L'extrait obtenu est dosé en tétraol par CLHP par rapport à une gamme d'étalonnage réalisée avec un échantillon authentique. Le dosage est exprimé en mg de tétraol par kg de feuilles séchées.

Les exemples présentés ont été réalisés sur une matière première dont la teneur en tétraol (en masse) expriméc par rapport à la feuille séchée est d'environ 900 mg/kg.

L'analyse CLHP est réalisée sur du matériel WATERS-MILLIPORE (référence Millenium) en condition isocratique (H₂O/CH₃CN: 75/25, 1 ml/mn) sur colonne Lichrospher 100 MERCK (5 µm). La quantité injectée est de 20 µl, l'intégration est réalisée à 230 nm.

Les conditions d'analyses CLHP sont identiques pour le dosage des feuilles et pour l'évaluation de la pureté du tétraol isolé lors des manipulations.

L'échantillon de référence ainsi que le tétraol extrait au cours des différentes manipulations présentées ont montré les mêmes caractéristiques spectrales que celles mentionnées dans la littérature (¹H RMN, ¹³C RMN, UV, IR, point de fusion ...).

### EXEMPLE 1: Extraction acétone/eau, cristallisation acétonitrile (1ère variante) (ne fait par partie de l'invention telle que revendiquée)

30 kg de feuilles séchées de Taxus baccata (titre en tétraol = 900 mg/kg) sont broyées finement puis extraites par 300 litres d'un mélange acétone/eau (70/30) dans un réacteur inox de 600 litres pendant 1 heure à température ambiante, sous agitation. Après décantation, le marc isolé est soumis à une deuxième extraction dans des conditions identiques à la première.

Après essorage sur essoreuse ROBATEL SLP munie d'une toile Nylon de 20 µm et rinçage du marc par 50 litres d'acétone, on obtient 605 litres d'un extrait hydroacétonique enrichi en tétraol. Cet extrait est concentré par évaporation jusqu'à un volume final de 10 litres environ.

Les 10 litres de phase aqueuse concentrée obtenue précédemment, sont soumis à deux extractions volume à volume par l'acétonitrile (ou par une seule extraction par 2 ou 3 volumes d'acétonitrile pour 1 volume de phase aqueuse). Les phases organiques sont réunies, puis le solvant est évaporé sous pression atmosphérique de manière à réduire le volume de solvant organique et le sécher par entrainement azéotropique, jusqu'à l'obtention d'une solution presque sèche titrant 1,5 à 2 % H₂O. Cette solution est alors reprise par 5 litres d'acétonitrile, filtrée puis de nouveau concentrée à 3 litres.

La phase organique concentrée est ensuite conservée à 4°C pendant 24 heures pour précipiter le tétraol.

Le précipité cristallin obtenu (20,8 g) après filtration et séchage à température contrôlée sous vide poussé se présente sous la forme d'une poudre blanche titrant plus de 90 % en 10-désacétylbaccatine III. Le rendement d'extraction par rapport à la quantité de tétraol initialement présente dans les feuilles est de 70 %.

### EXEMPLE 2: Purification du tétraol par lavage (pas selon l'invention telle que revendiquée)

10 g du produit obtenu précédemment sont dissous dans 200 ml d'acétate d'éthyle. La solution obtenue -est ensuite lavée deux fois par 70 ml d'une solution de carbonate de sodium à 2 % puis par de l'eau jusqu'à pH neutre.

La phase organique est séchée, puis évaporée sous pression atmosphérique, et on obtient 8,9 g de tétraol de pureté supérieure à 96 %.

### EXEMPLE 3: Extraction acétone / eau, cristallisation acétonitrile (2ème variante)

1 kg de feuilles séchées de Taxus baccata (titre en tétraol = 900 mg/kg) sont broyées finement puis extraites par 10 litres d'un mélange acétone / eau (70/30) dans un réacteur verre de 20 litres pendant 1 heure à température ambiante, sous agitation. Après décantation, le marc isolé est soumis à une deuxième extraction dans des conditions identiques à la première.

Après essorage sur toile Nylon de 20 µm et rinçage du marc par 1 litre d'acétone, on obtient 19,5 litres d'un extrait hydroacétonique enrichi en tétraol. Cet extrait est concentré par évaporation jusqu'à un volume final de 0,3 litres environ.

Les 0,3 litres de phase aqueuse concentrée obtenue précédemment, sont soumis à deux extractions volume à volume par l'acétonitrile (ou par une seule extraction par 2 volumes d'acétonitrile pour 1 volume de phase aqueuse). Les phases organiques sont réunies, puis on ajoute 25 g de NaCl pour relarguer 60 ml d'eau puis concentrées par entrainement azéotropique de l'eau à pression atmosphérique jusqu'à l'obtention d'une solution presque sèche titrant environ 2 % H₂O. Cette solution est ensuite reprise par 500 ml d'acétonitrile, filtrée puis concentrée sous vide jusqu'à un volume de 100 ml, puis enfin abandonnée à 4°C pendant 24 heures pour précipiter le tétraol.

On obtient 0,731 g de précipité cristallin après filtration et séchage à température contrôlée sous vide poussé se présente sous la forme d'une poudre blanche titrant plus de 90 % en 10-désacétylbaccatine III. Le rendement d'extraction par rapport à la quantité de tétraol initialement présente dans les feuilles est de 74 %.

### EXEMPLE 4: Extraction acétone / eau, cristallisation acétonitrile, relargage par CH₂Cl₂

30 kg de feuilles séchées de Taxus Baccata (titre tétraol = 900 mg/kg) sont broyées finement puis extraites par 300 litres d'un mélange acétone / eau (70/30) dans un réacteur inox de 600 litres pendant 1 heure à température ambiante, sous agitation.

Après décantation, le marc est isolé et soumis à une deuxième extraction dans des conditions identiques à la première.

Après essorage sur essoreuse ROBATEL SLP munie d'une toile Nylon de 20 µm et rinçage du marc par 50 litres d'acétone, on obtient 605 litres d'un extrait hydroacétonique enrichi en tétraol.

Cet extrait est ensuite concentré jusqu'à un volume de 10 litres environ.

Ce concentrat aqueux est soumis à deux extractions volume à volume par l'acétonitrile (ou par une seule extraction par 2 volumes d'acétonitrile pour 1 volume de phase aqueuse). Les phases acétonitrile regroupées sont concentrées à 10 litres. Après lavage de la phase acétonitrile par 10 litres d'eau, l'eau résiduelle est relarguée par addition de 10 litres de dichlorométhane. Le dichlorométhane est évaporé sur l'acétonitrile, puis la phase acétonitrile est concentrée à 3 litres puis conservée à 4°C pendant 24 heures pour précipiter le tétraol.

Le précipité cristallin obtenu (20,5 grammes) après filtration et séchage à température contrôlée sous vide poussé se présente sous la forme d'une poudre blanche. Le rendement d'extraction par rapport à la quantité de tétraol initialement présente dans les feuilles est de 68 %. La pureté est supérieure à 90 %.

Le surnageant du cristallisat est repris pour la cristallisation du tétraol restant en solution dans l'acétonitrile. Le surnageant obtenu précédemment est réduit à un volume de 600 ml, puis lavé par 600 ml d'eau. L'eau résiduelle est relarguée par l'ajout de 600 ml de dichlorométhane, que l'on évapore ensuite. La solution d'acétonitrile est ensuite conservée à 4°C pendant 24 heures pour précipiter le tétraol. Après filtration et séchage on récupère de 3 à 4 g supplémentaires de tétraol sous la forme d'une poudre blanche de pureté supérieure à 90 %. Le rendement d'extraction par rapport à la quantité de tétraol initialement présente dans les feuilles est alors de l'ordre de 80 %.

## Revendications

1. Procédé d'extraction et d'isolement de la 10-désacétylbaccatine III (tétraol) à partir de feuilles d'if, **caractérisé en ce qu'**il consiste à mettre en oeuvre les étapes suivantes :
- première étape d'extraction des feuilles d'if par un solvant polaire protique consistant en un mélange acétone / eau pour obtenir une phase aqueuse concentrée contenant le tétraol,
- deuxième étape d'extraction du tétraol de ladite phase aqueuse par de l'acétonitrile,
- troisième étape de relargage de l'eau résiduelle de la phase acétonitrile par addition d'un solvant chloré,
- dernière étape d'isolement du tétraol par évaporation partielle de la phase organique, cristallisation sélective et filtration.

2. Procédé selon la revendication 1, **caractérisé en ce que** les feuilles d'if sont séchées et/ou broyées.

3. Procédé selon l'une des revendications 1 ou 2 **caractérisé en ce que** les feuilles d'if sont extraites par macération, dans le solvant polaire protique pour une durée comprise entre 15 minutes et 6 heures, de préférence 1 heure.

4. Procédé selon l'une des revendications 1 à 3, **caractérisé en ce que** les feuilles d'if sont extraites à une température comprise entre 10°C et la température d'ébullition du solvant polaire protique, de préférence à température ambiante.

5. Procédé selon l'une des revendications 1 à 4, **caractérisé en ce que** le mélange acétone / eau est utilisé dans un rapport volumique jusqu'à 80/20, avantageusement voisin de 70/30.

6. Procédé selon l'une des revendications 1 à 5, **caractérisé en ce que** la phase aqueuse est concentrée dans un rapport volume initial / volume final pouvant aller jusqu'à 70.

7. Procédé selon l'une des revendications 1 à 6, **caractérisé en ce que** la cristallisation sélective du tétraol est réalisée en laissant reposer à froid la solution de tétraol dans le solvant de cristallisation, de préférence à une température comprise entre 0°C et la température ambiante, avantageusement inférieure à 10°C, encore plus avantageusement comprise entre 0°C et 5°C.

8. Procédé selon l'une des revendications 1 à 7, **caractérisé en ce que** la cristallisation sélective est précédée par une étape de séchage de la phase organique et/ou de relargage de l'eau résiduelle.

9. Procédé selon la revendication 8, **caractérisé en ce que** la phase organique est lavée par l'eau avant le séchage et/ou le relargage.

## Claims

1. Process for the extraction and isolation of 10-deacetylbaccatin III (tetraol) from yew leaves, **characterized in that** it consists in carrying out the following steps:
- first step of extracting the yew leaves with a protic polar solvent consisting of an acetone/water mixture in order to obtain a concentrated aqueous phase containing the tetraol,
- second step of extracting the tetraol from the said aqueous phase with acetonitrile,
- third step of releasing the residual water from the acetonitrile phase by adding a chlorinated solvent,
- last step of isolating the tetraol by partial evaporation of the organic phase, selective crystallization and filtration.

2. Process according to Claim 1, **characterized in that** the yew leaves are dried and/or ground.

3. Process according to either of Claims 1 and 2, **characterized in that** the yew leaves are extracted by maceration in the protic polar solvent for a period of between 15 minutes and 6 hours, preferably 1 hour.

4. Process according to one of Claims 1 to 3, **characterized in that** the yew leaves are extracted at a temperature of between 10°C and the boiling point of the protic polar solvent, preferably at room temperature.

5. Process according to one of Claims 1 to 4, **characterized in that** the acetone/water mixture is used in a volume ratio of up to 80/20, advantageously of close to 70/30.

6. Process according to one of Claims 1 to 5, **characterized in that** the aqueous phase is concentrated in an initial volume/final volume ratio which may be up to 70.

7. Process according to one of Claims 1 to 6, **characterized in that** the selective crystallization of the tetraol is carried out by allowing the solution of tetraol in the crystallization solvent to stand in the cold state, preferably at a temperature of between 0°C and room temperature, advantageously of less than 10°C, more advantageously still of between 0°C and 5°C.

8. Process according to one of Claims 1 to 7, **characterized in that** the selective crystallization is preceded by a step for drying the organic phase and/or for releasing the residual water.

9. Process according to Claim 8, **characterized in that** the organic phase is washed with water before drying and/or releasing.

## Patentansprüche

1. Verfahren zur Extraktion und Isolierung von 10-Desacetylbaccatin III (Tetraol) ausgehend von Taxusblättern, **dadurch gekennzeichnet, dass** es aus der Durchführung der folgenden Stufen besteht:
- erste Stufe der Extraktion von Taxusblättern durch ein polares protisches Lösungsmittel, das aus einer Mischung von Aceton/Wasser besteht, um eine konzentrierte wässrige Phase zu erhalten, welche Tetraol enthält,
- zweite Stufe der Extraktion des Tetraols der wässrigen Phase mit Acetonitril,
- dritte Stufe des Aussalzens des rückständigen Wassers der Acetonitril-Phase durch Zugabe eines chlorierten Lösungsmittels,
- letzte Stufe der Isolierung des Tetraols durch teilweise Eindampfung der organischen Phase, selektive Kristallisation und Filtration.

2. Verfahren nach Anspruch 1, **dadurch gekennzeichnet, dass** die Taxusblätter getrocknet und/oder zerrieben sind.

3. Verfahren nach einem der Ansprüche 1 oder 2, **dadurch gekennzeichnet, dass** die Taxusblätter durch Mazerierung in dem polaren protischen Lösungsmittel über eine Dauer von 15 Minuten bis 6 Stunden, vorzugsweise 1 Stunde, extrahiert werden.

4. Verfahren nach einem der Ansprüche 1 bis 3, **dadurch gekennzeichnet, dass** die Taxusblätter bei einer Temperatur zwischen 10°C und der Temperatur des Siedepunktes des polaren protischen Lösungsmittels, vorzugsweise bei Umgebungstemperatur, extrahiert werden.

5. Verfahren nach einem der Ansprüche 1 bis 4, **dadurch gekennzeichnet, dass** die Mischung Aceton/Wasser in einem Volumenverhältnis bis zu 80/20, vorteilhaft etwa 70/30, verwendet wird.

6. Verfahren nach einem der Ansprüche 1 bis 5, **dadurch gekennzeichnet, dass** die wässrige Phase in einem Verhältnis Anfangsvolumen/Endvolumen, das bis zu 70 betragen kann, konzentriert wird.

7. Verfahren nach einem der Ansprüche 1 bis 6, **dadurch gekennzeichnet, dass** die selektive Kristallisation des Tetraols durchgeführt wird, indem man die Lösung des Tetraols in dem Kristallisationslösungsmittel in der Kälte ruhen lässt, vorzugsweise bei einer Temperatur zwischen 0°C und Umgebungstemperatur, vorteilhaft unterhalb von 10°C und noch vorteilhafter zwischen 0°C und 5°C.

8. Verfahren nach einem der Ansprüche 1 bis 7, **dadurch gekennzeichnet, dass** der selektiven Kristallisation eine Stufe der Trocknung der organischen Phase und/oder der Aussalzung des rückständigen Wassers vorangeht.

9. Verfahren nach Anspruch 8, **dadurch gekennzeichnet, dass** die organische Phase vor dem Trocknen und/oder dem Aussalzen mit Wasser gewaschen wird.
